# EUROPEAN PATENT SPECIFICATION

(11) **EP 1 196 210 B1**
(45) Date of publication and mention of the grant of the patent: **17.08.2005**
(21) Application number: 00937100.6
(22) Date of filing: 09.06.2000
(51) Int. Cl.: A61M 5/30

(54) **NEEDLELESS SYRINGE**
NADELLOSE SPRITZE
SERINGUE SANS AIGUILLE

(30) Priority: 16.07.1999 GB 9916800
(43) Date of publication of application: 17.04.2002
(60) Divisional application: 05010246.6
(73) Proprietor: PowderJect Research Limited, Oxford OX4 4GA (GB)
(72) Inventor: KENDALL, Mark A. F., 4 Robert Robinson Avenue, Oxford OX4 4GA (GB); BROWN, Garry, 4 Robert Robinson Avenue, Oxford OX4 4GA (GB)
(74) Representative: Price, Nigel John King
(86) International application number: PCT/GB2000/002257
(87) International publication number: WO 2001/005455

(56) References cited:
- EP-A- 0 821 195
- WO-A-96/25190
- WO-A-97/47730
- US-A- 5 899 880

## Description

The present invention relates generally to a needleless syringe device for accelerating particles for delivery into target tissue of a subject.

The ability to deliver pharmaceuticals through skin surfaces (transdermal delivery) provides many advantages over oral or parenteral delivery techniques. In particular, transdermal delivery provides a safe, convenient and non-invasive alternative to traditional drug administration systems, conveniently avoiding the major problems associated with oral delivery (e.g. variable rates of absorption and metabolism, gastrointestinal irritation and/or bitter or unpleasant drug tastes) or parenteral delivery (e.g. needle pain, the risk of introducing infection to treated individuals, the risk of contamination or infection of health care workers caused by accidental needle-sticks and the disposal of used needles). In addition, transdermal delivery affords a high degree of control over blood concentrations of administered pharmaceuticals.

Recently, a novel transdermal drug delivery system that entails the use of a needleless syringe to fire powders (i.e. solid drug-containing particles) in controlled doses into and through intact skin has been described. In particular, US Patent No. 5,630,796 to Bellhouse et al. describes a needleless syringe that delivers pharmaceutical particles entrained in a supersonic gas flow. The needleless syringe can be used for transdermal delivery of powdered therapeutic compounds and compositions (e.g. drugs, vaccines, etc.), for delivery of genetic material into living cells (e.g. gene therapy) and for the delivery of biopharmaceuticals to skin, muscle, blood or lymph. The needleless syringe can also be used in conjunction with surgery to deliver particles to organ surfaces, solid tumours and/or to surgical cavities (e.g. tumour beds or cavities after tumour resection). In theory, practically any pharmaceutical agent that can be prepared in a substantially solid, particulate form can be safely and easily delivered using such devices.

One needleless syringe described in Bellhouse et al. comprises an elongate tubular over-expanded converging-diverging nozzle having a rupturable membrane initially closing the passage through the nozzle and arranged substantially adjacent to the upstream end of the nozzle. Particles to be delivered are disposed adjacent to the rupturable membrane and are delivered using an energising means which applies a gaseous pressure to the upstream side of the membrane sufficient to rupture the membrane and produce a supersonic gas flow (containing the pharmaceutical particles) through the nozzle for delivery from the downstream end thereof.

Transdermal delivery using the needleless syringe described in Bellhouse et al. is carried out with particles having an approximate size that generally ranges from between 0.1 and 250 µm. For drug delivery, an optimal particle size is usually at least about 10 to 15 µm (the size of a typical cell). For gene delivery, an optimal particle size is generally substantially smaller than 10 µm. Particles larger than about 250 µm can also be delivered from the device, with the upper limitation being the point at which the size of the particles would cause untoward damage to the skin cells. The actual distance which the delivered particles will penetrate depends upon particle size (e.g. the nominal particle diameter assuming a roughly spherical particle geometry), particle density, the initial velocity at which the particle impacts the skin surface, and the density and kinematic viscosity of the skin. In this regard, optimal particle densities for use in needleless injection generally range between about 0.1 and 25 g/cm³, preferably between about 0.8 and 1.5 g/cm³, and injection velocities generally range between about 100 and 3000 m/sec. These particle size and density ranges are also appropriate to the present invention.

There are two distinct phases of gas flow that occur in the device. The first phase is associated with the shock waves produced upon rupturing of the membrane and is called "the starting process" (or "starting transient"). The second regime of flow occurs upstream of the shock and expansion waves associated with starting process and is called quasi-steady nozzle flow.

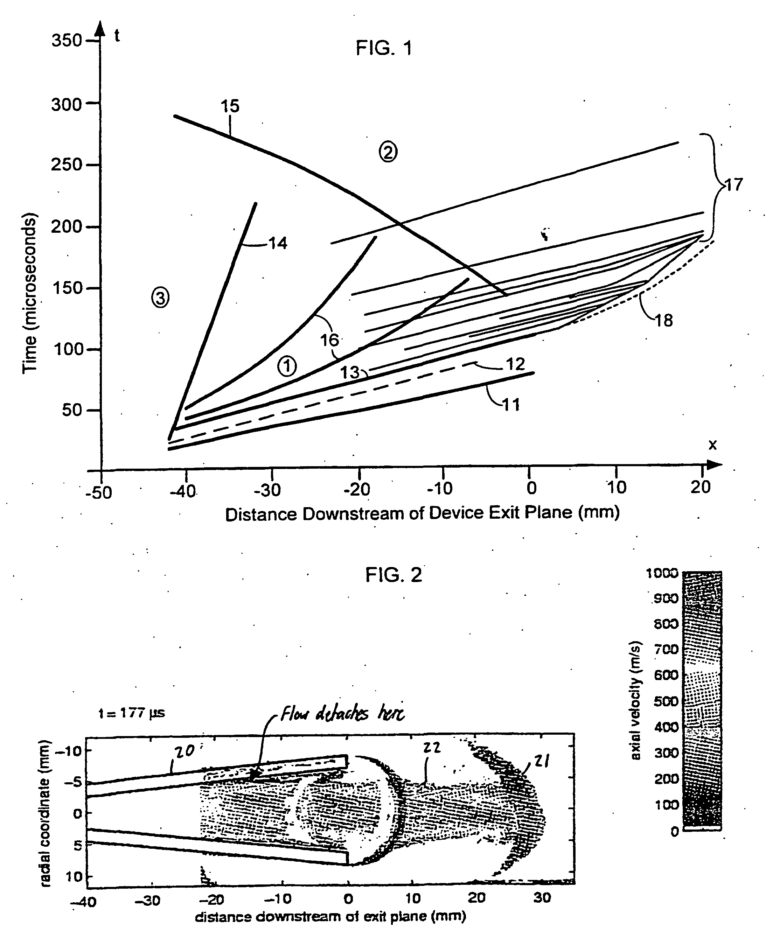

As discussed in Bellhouse et al, it was considered that the particle velocity depended upon the flow in the starting process. The starting process is generated by a sudden impulse change in pressure within the divergent nozzle, and in the Bellhouse et al device is initiated at the throat of the nozzle. This is shown by the space-time (*x*-*t*) diagram of Figure 1. This Figure shows the distance downstream (positive values of *x*) and upstream (negative values of *x*) of the nozzle exit plane (i.e. the distal end of the nozzle) along the abscissa and shows time on the ordinate. The time starts when the device is actuated. After rupturing of the membrane (which is located at around *x*=-50) at *t*=0, a steep front of high pressure (a shock wave 11) sweeps downstream along the length of the nozzle. This is closely followed by the so-called "contact surface" 12.

The contact surface 12 is the boundary between the gases that were previously separated by the membrane. It is well acknowledged that the gases do not mix appreciably at this boundary so the effect is one of the driver gas (the gas upstream of the membrane before rupturing) "pushing" the driven gas (the gas downstream of the membrane before rupturing) out of the nozzle like a piston, with the contact surface 12 being analogous to the face of the piston. The contact surface 12 is closely followed by a secondary shock wave 13. The secondary shock wave 13 is followed by a series of oblique shock fronts 16 within a starting process (region 1 in Figure 1) with large variations in gas density and velocity (and therefore particle velocity). The starting process region 1 is substantially bounded by the shocks 11, 14 and 15; shock front 15 is mentioned further below.

The starting process is followed by a regime of quasi-steady flow (in region 3). The quasi-steady flow is clean, that is to say substantially free of shock waves such that the velocity at a given point changes slowly enough with time to be accurately modelled by steady flow non time-varying equations. Quasi-steady flow is thus different from truly steady flow in which the Mach number at a given point does not change over time, and unsteady flow in which the Mach number at a given point varies, and the flow is governed by unsteady equations. Both the starting process and quasi-steady flow are terminated by an oblique shock front 15 that is swept upstream of the nozzle exit, as a result of over-expanded nozzle operation. This shock marks the boundary of region 2. As is mentioned in Bellhouse et al, it was thought that the particles, being initially positioned on, or very near to, the rupturable membrane, travelled with the contact surface 12 between the fronts of the primary and secondary shock waves 11,13. In the light of investigations by the present inventors, this view is now believed to be over-simplistic and (as is discussed later) the gas-particle flow in such prior art devices is more complicated with groups of particles being accelerated by different mechanisms. It is still true that the starting process is critical to the acceleration of a proportion of the particles in prior art devices. In contrast to this, the essence of the present invention is based on the idea of trying to avoid entraining the particles in the starting process.

Previous devices have utilised over-expanded nozzles which have an exit cross-sectional area *A*_{*e*} greater than the exit cross-sectional area of a correctly-expanded nozzle *A*_{*correct*}. Over-expanded operation occurs when the ratio *P*_{*tot*}/*P*_{*e*} of the total pressure *P*_{*tot*} to the ambient exit pressure *P*_{*e*} is insufficient for a correctly expanded supersonic flow for a given nozzle area ratio *A*_{*1*}/*A*_{*e*} (where *A*_{*1*} is the minimum diameter in the system). An overexpanded nozzle was used in prior art devices because, in order to obtain an adequate spread of payload on the target, it was thought that a large exit area was required. However, experiments have shown that using an over-expanded nozzle leads to flow non-uniformities such as oblique (or normal) quasi-stationary shock waves in the flow which serve to detach the flow from the nozzle walls. This flow accelerates the particles in a separated jet. Thus, surprisingly, it has been found that using a larger exit area does not necessarily increase the useful target area and in fact often causes the flow to detach resulting in a central jet core forming and a consequent low payload spread. This is undesirable and is known as "jetting".

Furthermore, devices of the prior art (such as those described in Bellhouse et al) utilise a convergent nozzle portion downstream of the particle-containing cassette. This portion acts as the interface between the relatively large membrane diameter and the relatively small nozzle throat diameter. The chosen throat diameter is controlled by the desired maximum choked mass flow rate through the device and the chosen membrane diameter is controlled by the need to be easily able to manufacture the cassette and hold the dose of particles required. Thus, upon actuation, the particles are forced through a constriction in the device. It is thought that this may increase particle-wall attrition and reduce the particle size and thereby undesirably affect the acceleration and penetration characteristics of the particles.

Experimental research sponsored by the present applicant has shown that the prior art devices produce two distinctive types of particle behaviour. Results from time resolved DGV (Doppler Global Velocimetry) measurements are shown in Figure 2, ( see Kendall MAF, Quinlan NJ, Thorpe SJ, Ainsworth RW and Bellhouse BJ (1999), "The gas dynamics of a high speed needle-free drug delivery system", International Symposium on Shock Waves 22, Imperial College, London, July 19-23 and Quinlan NJ, Thorpe SJ and Ainsworth RW (1999), "Time-resolved Doppler global velocimetry of gas-particle flows in transdermal powder drug delivery", 8^{th} Int. Conf. On Laser Anemometry- Advances and Applications, 6-9 Sept, University of Rome, Italy. This shows a cross-section of the divergent portion of a nozzle 20 and gives the instantaneous speed of the particles at a time of 177 µs after rupture of the membrane (not shown).

As can be seen, the leading particles 21 are delivered in a wide cloud at a typical velocity of 200 - 400 m/s. A narrower quasi-steady stream of particles 22 follows the leading cloud at 650 - 800 m/s; it should be noted that the white circular image centred on the plane of the nozzle exit together with the dark shadow on its right hand boundary as drawn is an artefact produced by this measurement technique. The leading particles are associated with the transient starting process in the gas flow, while the high speed particles are entrained in the quasi-steady nozzle flow. As has been mentioned, the nozzle in this prior art device is over-expanded which means oblique shocks will be present in the nozzle. The detachment of the high velocity particle stream from the nozzle walls is a direct consequence of the shock induced separation of the nozzle gas flow due to these shocks. Referring again to Figure 1, the gas flow has been broadly categorised into three flow regimes:
i) The Starting Process (region 1)
ii) Shock-Processed Flow (region 2)
iii) Quasi-Steady Supersonic Flow (region 3)

The particle trajectories 17 are also shown in Figure 1. As can be seen, a significant proportion of the particles are accelerated within the starting process but then decelerate as they reach the secondary shock wave 13 and contact surface 12. A cloud front 18 of particles is seen to decelerate as it leaves the nozzle, the nozzle exit being represented by *x*=0 These particles are those entrained with the initial 200 - 400 m/s cloud attached to the nozzle wall. By nature, the starting process creates a flow having large variations in axial gas velocity and density. These are thought to be the two most important parameters for particle acceleration. There are also large variations in gas velocity and density radially. This flow regime is therefore considered unsuitable for drug delivery if uniform velocities and distributions are required. Another fraction of particles do not reach the secondary shock 13 but are processed firstly by the oblique shocks 16 within the starting process (in region 1) and then the upstream moving oblique shock 15 which defines the separated flow within region 2. The final component of the particle payload is accelerated within the quasi-steady flow (region 3, particle trajectories not shown in Figure), before being separated by the quasi-stationary shock front 14 (defined in region 2). This acceleration path leads to the highest particle velocity of 850 m/s confined to a separated jet of approximately 9 mm diameter.

It seems, contrary to previous beliefs, that the starting process, rather than being the chief accelerator of the particles, actually acts as an impediment to high velocity particles. The particles which exit initially in a large cloud seem to act as a barrier to particles entrained in the subsequent quasi-steady flow resulting in lower overall particle velocities, which can be undesirable.

The present invention arises from the idea that, if one can prevent the particles from being entrained in the starting process flow, then substantially all of the particles will be entrained in the subsequent quasi-steady supersonic flow, resulting in higher and more uniform particle velocities. The present invention also alleviates the problem of "jetting" by using a substantially correctly expanded nozzle and the problem of particle attrition by dispensing with a convergence downstream of the membrane.

US 5,899,880 discloses the pre-characterising portion of claim 1.

Accordingly, there is described a method of accelerating a dose of particles in a needleless injection device having a driver chamber and a duct section downstream of said driver chamber, the method comprising:
opening closure means located between said driver chamber and said duct section;
producing a primary shock wave travelling in a downstream direction in said duct section;
establishing a substantially quasi-steady flow of fluid in said duct section upstream of said primary shock wave; and
entraining and accelerating substantially all the dose of particles in said substantially quasi-steady flow for the duration of time that said particles are in said duct section.

A starting process may be created when the primary shock wave reaches the downstream end of the duct. A secondary shock wave may also be produced behind the primary shock wave and the quasi-steady flow is preferably established upstream of the secondary shock wave, and continues after the secondary shock wave has passed out of the device.

The present invention includes a needleless injection device comprising:
a driver chamber arranged, in use, to contain a charge of pressurised gas;
a duct section connected to said driver chamber to receive gas therefrom;
closure means for preventing the flow of gas from said driver chamber to said duct section until said closure means is opened; and
a dose of particles positioned within the device in the region of said closure means;
said device being so constructed and arranged that upon opening of said closure means, a primary shock wave is produced to travel along said duct section in a downstream direction and a substantially quasi-steady gas flow is established in said duct section upstream of said primary shock wave, said dose of particles being substantially wholly entrained in said substantially quasi-steady flow to be accelerated thereby and expelled from the device.

The driver chamber may be pre-charged with gas or could be connected to a source of gas operable to charge the driver chamber with pressurised gas. The driver chamber may be constituted by a constant area tube or may have a convergence at its downstream end.

The duct section is advantageously of a constant cross-sectional area and the particles are usefully positioned upstream of the closure means.

Preferably, there is no convergent portion downstream of the closure means and there is a divergent portion downstream of the duct section. When such a divergent portion is provided, the shock wave initiates a transient starting process upon reaching it and this transient process is followed by a quasi-steady supersonic flow in the divergent portion.

The purpose of the divergent portion is to further accelerate the entrained particles in a controlled manner. The divergent portion preferably has an area ratio such that flow there through is substantially correctly expanded and may also be contoured to prevent oblique shock waves forming in the divergence and/or to provide a uniform distribution of particles.

A further closure means may be provided and this or the first said closure means may comprise a rupturable membrane. When two closure means are used, the particles are advantageously positioned between them and each closure may have the same or different opening pressures.

Embodiments of needleless syringe device in accordance with the present invention will now be described, by way of example only, with reference to the accompanying drawings in which:
Figure 1 shows schematically an x-t diagram which describes the flow regimes present in a prior art device similar to the ones described in Bellhouse et al;
Figure 2 shows a cross-sectional view of the nozzle and the instantaneous axial velocity of particles exiting the above-mentioned prior art device after 177µs of flow;
Figure 3 shows schematically an *x*-*t* diagram which describes the flow regimes present in a device according to a first embodiment of the present invention;
Figure 4 shows a schematic cross-sectional side elevation of a target surface and an impingement region;
Figure 5 shows a needleless injection device according to a first embodiment of the present invention in schematic cross-sectional side elevation;
Figure 6 shows a part of a schematic *x*-*t* diagram which describes the behaviour of the starting process in a device according to a second embodiment of the present invention when the gas in region 2 is subsonic and in region 3 is supersonic;
Figure 7 shows a part of a schematic *x-t* diagram which describes the behaviour of the starting process in a device according to a third embodiment of the present invention when the gas in regions 2 and 3 is subsonic;
Figures 8a and 8b are schematic cross-sectional (before and after) side elevations of the membrane region of a duct section of a fourth embodiment of a needleless injection device and illustrate a further aspect of the invention wherein the duct section has an enlarged duct portion to keep a more constant cross-sectional area after membrane bursting;
Figure 9 shows a fifth embodiment which is a modification to the Figure 5 embodiment wherein the driver chamber has a larger cross-sectional area than the duct section, only part of the device being shown;
Figures 10a, 10b and 10c are a sequence of schematic cross-sectional side elevations of the membrane region of a sixth embodiment of a needleless injection device and show another aspect of the invention relating to the creation of a mixed gas-particle cloud between two closures in a driver chamber;
Figures 11a, 11b, 11c, 11d and 11e show a seventh embodiment which involves a similar sequence to Fig. 10 except the rupture pressures and timing of the particle entrainment are different;
Figures 12a, 12b and 12c are a sequence of schematic cross-sectional side elevations of the membrane region of an eighth embodiment and show the use of a transfer duct and a separate rupturable membrane to create a mixed gas-particle cloud between two closures in the driver chamber; and
Figures 13a, 13b, and 13c are a sequence of schematic cross-sectional side elevations of the membrane region of a modification of the eighth embodiment in which the transfer duct is positioned in the upstream closure means.

### Embodiment 1

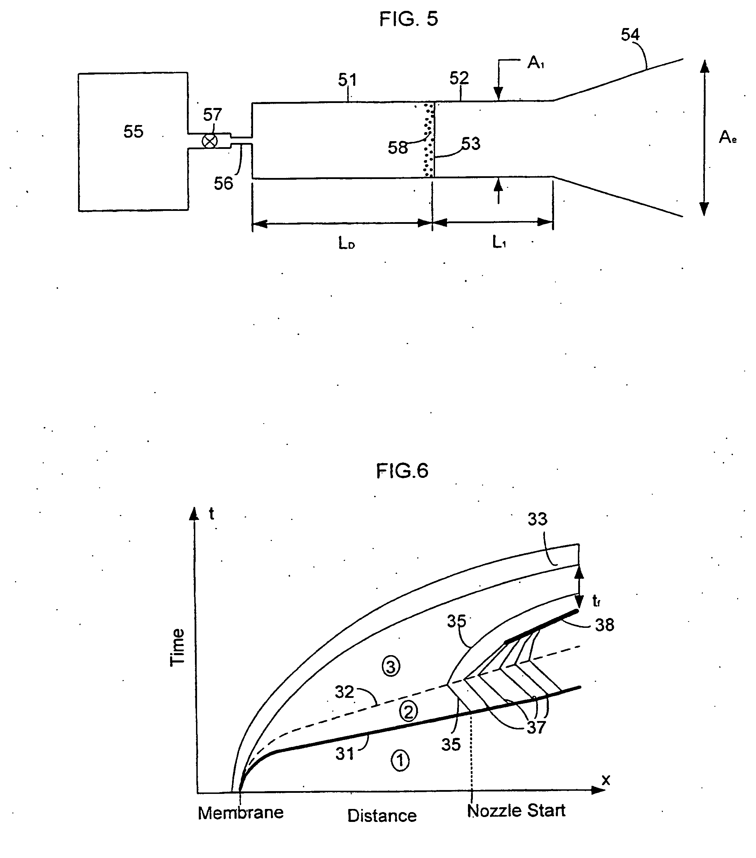

The first embodiment of the invention is an air powered, disposable device and is shown schematically in Figure 5. The device could, however, be reusable and/or powered by a fluid other than air, for example helium, nitrogen or a mixture of gases. The selection of gas can be used to tune the performance of the device. Different gases or gas mixtures provide different quasi-steady gas velocities in the same device and so the target particle velocity can be closely controlled by an appropriate driver gas selection.

The device comprises an elongate tubular driver chamber 51 attached to a cylindrical duct section (or shock tube) 52 of the same diameter as the driver chamber 51. In this embodiment, each tube has a 6mm diameter, but in general the diameters can be different to one another and can be of any practical size.

In this embodiment, the driver chamber 51 has a length *L*_{*D*} of 65 mm and the duct section 52 has a length *L*_{*1*} of 30 mm. Other lengths are possible, and in fact the determination of the lengths is thought to be important in influencing the performance of the device (see later).

At the interface between the driver chamber 51 and duct section 52 is a rupturable membrane 53. The membrane 53 is of the type disclosed in Bellhouse et al and typically ruptures with a pressure difference across it in a range from around 5 to 20 bar, preferably 10 to 15 bar. The rupture pressure is an important device parameter but other rupture pressures could also be used depending on the desired results. Control of the rupture process can be important for mixing and flow uniformity and may be enhanced by the prior indentation or scoring of the membrane, preferably along radial lines along which the rupture will propagate. This provides for more symmetrical membrane opening which in turn can provide a more symmetrical particle distribution at the target plane.

The downstream end of the duct section 52 is provided with a contoured divergent nozzle 54 in this embodiment. The nozzle 54 has an area ratio *A*_{*e*}/*A*_{*1*} such that correctly expanded flow is established within it when the membrane 53 has ruptured and the driver chamber 51 discharges. In practice this ratio (*A*_{*e*}/*A*_{*1*}) could range from 1 to 50. As drawn, the contoured nozzle 54 could is of conical shape with a half angle which is not so steep as to cause flow separation. Half angles up to 15° could be used in practice, and it has been found that 6° operates satisfactorily. The divergent nozzle 54 could take other forms and, in fact, is not essential to the present invention.

The driver chamber 51 is connected at its upstream end to a reservoir 55 of pressurised gas (in this case air) by a small diameter bleed hole 56. Other gases or gas mixtures which are sterile and easily obtainable such as helium, nitrogen, argon or CO₂ are also suitable. The gas pressure in the reservoir 55 should be sufficient for the gas to be able to pass into the driver chamber 51 and rupture the membrane 53. In this embodiment the gas pressure is 60 bar but could be higher or lower depending on the membrane rupturing pressure. Also, other energising means (such as explosive charges) could be used to discharge gas into the driver chamber 51.

The reservoir 55 may be connected to the bleed hole 56 in a standard way (such as with a valve 57 as shown in the Figure) such that a flow of gas from the reservoir 55 to the driver chamber 51 can be initiated on demand. In this embodiment the bleed hole 56 has a diameter of 0.4 mm. This effectively de-couples the reservoir 55 and driver chamber 51 during the period of device operation (for an explanation of de-coupling, see below). However, other sizes of bleed hole could be used (for e.g. from 0.1 mm to 5 mm). When larger holes are used, total decoupling would not be established and the total pressure *P*_{*tot*} in the driver chamber 51 would be able to rise as the device is actuated (with de-coupling, the total pressure remains constant).

As a further alternative, the driver chamber 51 could be pre-charged with pressurised gas and the reservoir 55 omitted. In such an arrangement, the membrane 53 could be punctured mechanically to actuate the device.

The particles 58 to be accelerated are in this embodiment initially located in the driver chamber 51 in the region of the rupturable membrane 53. The particles 58 do not necessarily have to be initially located adjacent to the membrane 53. If they are located anywhere in the driver chamber 51 initially, they will not be entrained in the starting transient and so the invention should still operate. Also, the particles 58 could be located adjacent to the downstream side of the membrane 53 and the device should still work.

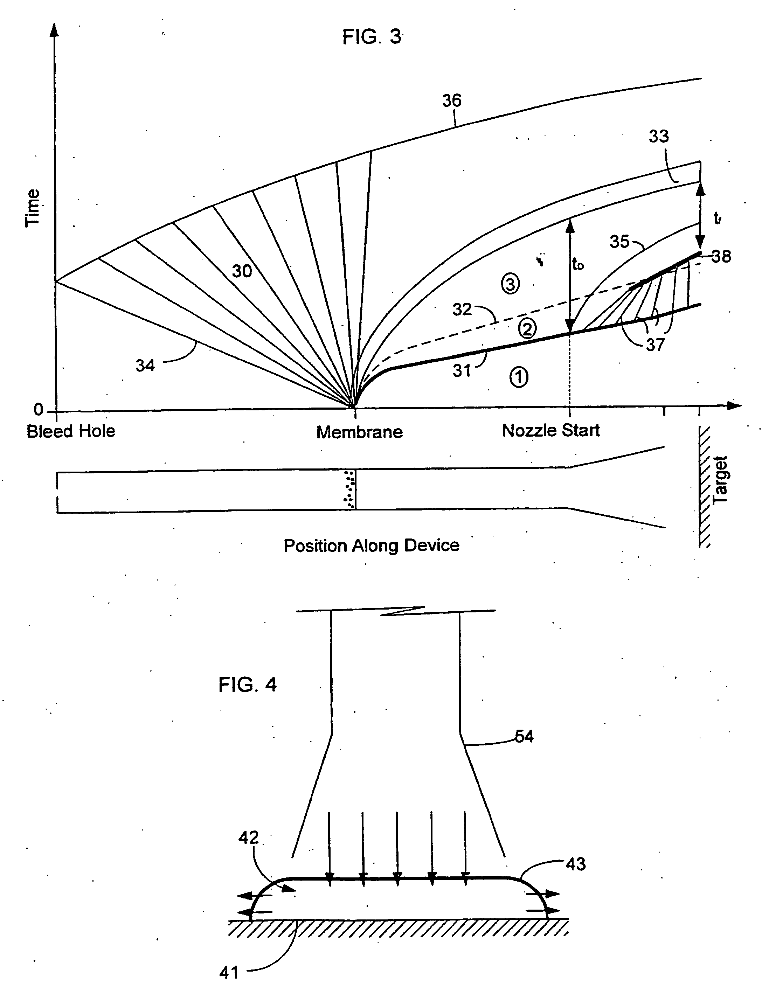

The functioning of this device is shown schematically by the *x*-*t* diagram in Figure 3, with *t*=0 corresponding to membrane rupture. When the reservoir valve 57 is opened, gas flows from the reservoir 55 to the driver chamber 51 via the bleed hole 56 until the membrane rupture pressure is reached in the driver chamber 51. Thus, upon rupturing of the membrane 53, a shock 31 is generated which travels down the duct section 52 in the downstream direction. After a characteristic shock formation distance, the shock 31 travels ahead of the contact surface 32 at a constant speed. The contact surface 32 follows closely behind the shock 31 and the particles 33 follow behind that. Three flow regions can be identified:
i) Quiescent gas ahead of the shock wave 31 (region 1)
ii) Gas between the shock 31 and the contact surface 32 (region 2)
iii) Gas between the contact surface 32 and the particles 33 (region 3)

The distance between the particles 33 and the contact surface 32 increases initially with time because of the slower acceleration of the particles compared with the gas. The function of the duct section 52 is to provide a distance over which the shock 31 and the contact surface 32 can form so that the separation between the shock 31 (which will initiate the starting process at the transition between the duct 52 and the divergent nozzle 54), i.e. the "Nozzle Start" position in Fig. 3, and the particles 33 increases. The instantaneous delay time *t*_{*D*} (the time between the starting process initiating and the particles reaching the divergence 54) is a function of the particle size and gas type, whereby larger and denser particles are delayed more.

Simultaneously to the above, a first *(u-a)* expansion wave 34 moves at a constant velocity (initially the speed of sound in the gas, *a*) from the location of the ruptured membrane 53 in the upstream direction until it reaches the bleed hole 56. Here it is reflected back as a *(u*+*a)* wave 36 in the downstream direction where it accelerates until it eventually exits through the nozzle 54. The gas velocity in the downstream direction is denoted by *u* and the local speed of sound in the gas is denoted by *a*. Further *(u-a)* expansion waves are created and the result is unsteady expansion fan 30 shown in Figure 3.

As the shock 31 moves through the tube in region 2, it serves to process the quiescent gas in region 1 to be in region 2 and heats up the this gas it processes, increasing its temperature and density. This is the so-called "shock-heating" process.

When the shock 31 reaches the start of the divergent nozzle 54, the starting process is initiated and a second *(u-a)* wave 35 is produced at the transition between the constant area 52 and divergence 54. In the embodiment of Figure 3, the driver gas is such and the pressure is sufficiently high for the Mach number of the gas in both regions 2 and 3 to be greater than 1. This second *(u-a)* wave 35 travels relatively slowly along the nozzle 54 in the downstream direction and accelerates once the contact surface 32 has overtaken it. This occurs because the gas ahead of the contact surface 32 in region 2 has been shock-heated by the passing of the shockwave and so has a higher temperature and speed of sound than the gas behind the contact surface 32 in region 3. The gas in region 3 has been cooled by the expansion wave 34. Thus, when the second *(u-a)* wave 35 is overtaken by the contact surface 32, it speeds up in the downstream direction because the value of *a* drops suddenly (whereas the value of the velocity, *u*, is matched across the contact surface 32). The shock heating and expansion cooling processes are therefore beneficial in containing and in accelerating the starting process out of the device.

As the shock 31 propagates in the divergent section there is a complex system of *(u-a)* waves established. This system of waves 37 is downstream of the *(u-a)* wave 35 in this example. The waves 37 are required to match the flow upstream of the shock 31 and the quasi-steady supersonic flow in the divergent nozzle 54. This system of waves 37 generally coalesces to form a secondary shock wave 38. As shown in Figure 3 the second *(u-a)* wave 35 and the downstream system of waves 37 and 38 may pass into region 3.

The length *L*_{*1*} of the duct section 52 is chosen so that the bulk of the particle cloud 33 is accelerated in the diverging duct 54 by a quasi-steady supersonic flow. This can be achieved by ensuring that the secondary shock 38 precedes, and leaves the diverging duct ahead of, the particle cloud. Quasi-steady flow is a substantially steady flow and in particular in this application is a flow substantially free of shock waves. Expansion waves (such as *(u-a)* wave 35) may be present in this quasi-steady flow.

In other words, the device is arranged so that the final delay time *t*_{*f*} (the time between the secondary shock 38 and the head of the particle cloud 33) is positive.

Further, the length *L*_{*1*} of the duct section 52 is also important for the reason that, the longer it is, the more the particles 33 are accelerated and approach the gas velocity. The gas in region 3 has a uniform density and velocity so the particles 33 experience a uniform acceleration due to the difference between their velocity and that of the gas.

A long length *L*_{*1*} would theoretically lead to particle velocities close to the gas velocity. However, in practice, increasing the length *L*_{*1*} to beyond a certain point will give diminishing returns due to shock attenuation and the contact surface 32 moving closer to the shock wave 31 as a result of boundary layer growth at the duct walls. There is therefore an optimum duct length of *L*_{*1*} which also depends on the other parameters (such as the driver gas species and pressure), and other constraints of the system.

The length *L*_{*D*} of the driver chamber 51 is chosen so that the particles 58 have passed out of the device before the reflected expansion wave 36 passes out of the device. In other words, the length is preferably chosen so that the reflected expansion wave 36 nominally does not overtake the bulk of particle cloud 33. This length ideally needs to be longer if light gases are used in the driver chamber (e.g. helium) which have a higher speed of sound. Thus, the boundary in time between the point where the shock wave 38 arrives at the divergent nozzle exit (effectively terminating the starting process) and the point where the reflected first *(u-a)* wave 36 arrives at the nozzle exit delimit a regime of clean, quasi-steady flow. Substantially all of the particles 58 are entrained in this regime of clean flow (otherwise termed as the particle "delivery window").

Upon actuation, the bleed hole 56 causes the driver chamber 51 to be filled gradually until the membrane rupture pressure is reached. The bleed hole 56 (which could be constituted by an orifice plate) serves to ensure that during the discharge process, a negligible amount of gas is able to escape from the reservoir 55 into the driver chamber 51. The bleed hole 56 therefore effectively creates an end-wall condition and has the effect of de-coupling the reservoir 55 from the flow system. Thus, the static pressure *P*_{*static*} in the driver chamber 51 remains substantially constant throughout the time the particles are accelerated. In this embodiment, the static pressure *P*_{*static*} in the driver chamber 51 is the membrane rupture pressure. The atmospheric (or ambient) exit pressure initially at the nozzle exit is denoted by *P*_{*e*}. The ratio *P*_{*3*}/*P*_{*e*} (*P*_{*3*} is the static pressure in region 3) is matched via analytical equations to the ratio *A*_{*1*}/*A*_{*e*} to ensure a quasi-steady correctly expanded flow through the nozzle section 54. Since the total pressure is constant, the nozzle 54 will be correctly expanded for the duration of the particle delivery window resulting in attached flow for substantially the whole actuation period. The flow is therefore clean and attached for the period in which particles 58 are entrained. The above description relates to a correctly expanded nozzle. However, the system is robust and experiments have shown that substantially clean and attached flow can also be obtained with an under-expanded nozzle or even a slightly overexpanded nozzle. A correctly expanded nozzle is preferred though.

The bleed hole 56 also makes the device easier to silence because it restricts gas flowrate from the reservoir 55.

Ensuring that the particles 58 are entrained in the quasi-steady flow is believed to provide a further advantage. When a flow impinges on a flat area 41 (in this case the skin or other tissue), an "impingement region" (see Figure 4) is set up. This region comprises a stagnation bubble 42 which serves to reduce the speed of the particles 58 as they approach the surface of the skin 41. Ideally, the nozzle exit is maintained at a predetermined distance from the target plane by a spacer (not shown in Figure 4). The quasi-steady flow expanded from region 3 within the divergent duct is a supersonic flow and it is slowed down in the impingement region by a shock wave 43. With the correctly expanded divergent nozzle, this supersonic flow forms a parallel jet at the exit and thus creates an essentially normal shock 43 in the impingement region. This controlled impingement region ensures that the drug particles 58 maintain uniform velocities from the jet centreline to the outer edges as they decelerate after passing through the shock and before impacting the skin or tissue target.

### Embodiment 2

In the above embodiment, the gas flow in both regions 2 and 3 is supersonic (i.e. it has a Mach number, *M*>1). However it is to be noted that the device also works when the gas in region 2 has a Mach number of less than 1. In this case, the nature of the starting process is altered as is shown in Figure 6, where identical reference numerals correspond to similar features. Here, the *(u-a)* wave 35 initially travels upstream (because *u* is now smaller than *a*). This *(u-a)* wave 35 and subsequent *(u-a)* waves 37 are reflected and transmitted at the contact surface 32 as *(u*+*a)* and *(u-a)* waves respectively (only the transmitted *(u-a)* waves are shown in Figure 6). The complex system of waves 37 coalesces to form again a secondary shock 38.

Therefore the net effect of *M*<1 in region 2 is a shift in the time of arrival at the nozzle exit of the secondary shock 38 (which effectively terminates the starting process). This can be seen by comparing Figures 3 and 6 wherein shock 38 arrives later in Figure 6 than in Figure 3, giving a reduced value of *t*_{*f*} in Figure 6. This simply changes the duration of the delimited regime of clean flow (the delivery window) between the arrival of the secondary shock 38 and the arrival of the first reflected *(u-a)* wave 36 (not shown in Figure 6).

### Embodiment 3

A further possibility for device operation occurs when the flow in region 3 has a Mach number of less than 1.

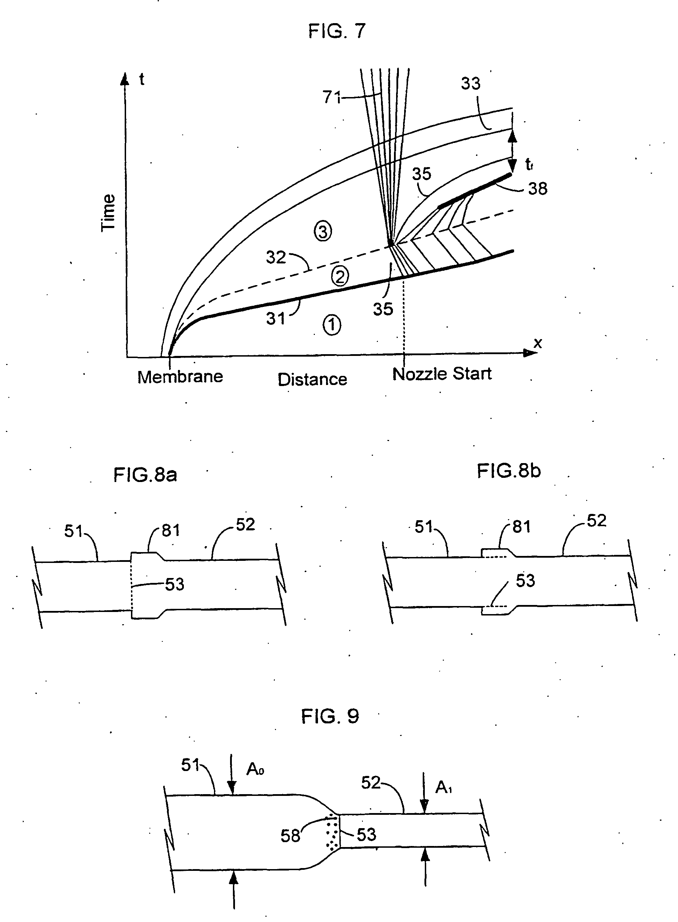

If the driven gas is air and the driver duct 51 is initially provided with:
- air (or nitrogen, or other gases with comparatively high molecular weights) with a closure means breaking at a sufficiently low pressure, or;
- a gas species other than air with a higher speed of sound than air; then it is possible for the flow in region 3 to have a Mach number of less than 1 (see Figure 7). In this third embodiment, the gas in region 3 will expand to Mach 1 via a second unsteady expansion fan 71 initiated at the start of the nozzle divergent section 54, provided that the driver total pressure is above a critical value. This expansion fan 71 brings the gas to sonic velocity at the upstream end of the divergent section 54. This sonic gas then expands quasi-steadily as a supersonic flow in the divergent section, as described in the earlier two embodiments. Furthermore, the starting process described in the earlier embodiments is present here, with the details dependent upon whether the flow in region 2 is greater or less Mach 1. The flow in region 2 is subsonic in Figure 7. Thus a delivery window of quasi-steady flow, substantially correctly expanded and uniform, is achieved within which the drug particle payload is nominally entrained.

### Embodiment 4

It has been found that the device is quite sensitive to the membrane opening area. Thus, it is desirable that the membrane 53 when ruptured (or any other suitable closure when opened) should present an area substantially identical to the area of the duct section 52. An example of apparatus to achieve this is shown in Figures 8a and 8b. Figure 8a shows the situation before rupture. An annular channel 81 is disposed adjacent to the downstream side of the membrane 53 (shown dashed in Figure 8 but in fact it is non-porous) so that when the membrane 53 ruptures, the area presented to the gas flow is substantially constant (see Figure 8b). If the area presented is smaller, a constriction occurs in the flow resulting in undesirable gas dynamics such as the creation of a steady expansion and flow perturbations.

### Embodiment 5

In the above embodiments, the driver chamber 51 is shown as having the same area as the duct section 52. However, the driver chamber 51 could be constructed so as to have a larger area than the duct section 52. This is shown in Figure 9. Such a construction causes a weaker unsteady expansion fan 30 and therefore a weaker reflected *(u*+*a)* wave 36. The weaker expansion waves caused by larger driver chamber cross-sectional area cause a smaller disruption to the acceleration of the particles 58 should these waves catch up to some of the particles before entry into the skin tissue or target.

Further, this construction makes the device less sensitive to variations in the membrane opening area. It is to be noted that the driver chamber area *A*_{*0*} is preferably not less than the duct section area *A*_{*1*} because this would result effectively in a divergence at the membrane. This would create waves at the point where the particles start and so might be unlikely to allow the starting transient to pass out of the nozzle before the particles 58 are entrained in the gas flow.

### Embodiment 6

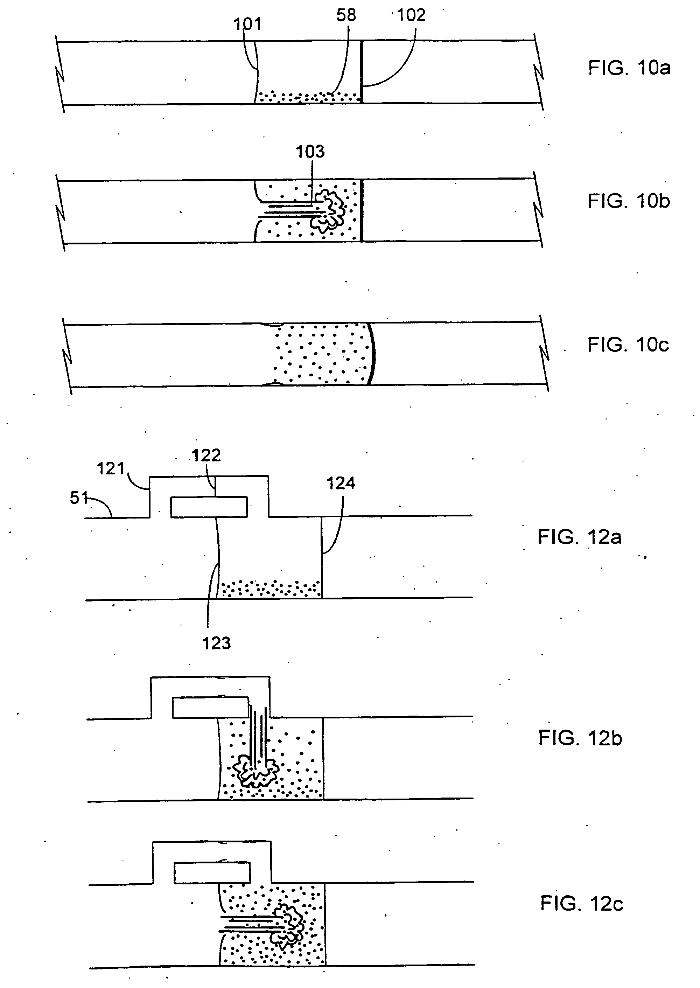

Another possible aspect of the invention to enhance particle mixing will now be described. Figure 10a shows a device having two membranes(101,102). The particles 58 are initially located between the two membranes in the driver chamber 51. The membranes are constituted so as to have different rupturing pressures, the upstream membrane 101 having a lower rupturing pressure than the downstream membrane 102. As the driver chamber 51 is filled and the upstream rupturing pressure is reached, the first membrane 101 ruptures (see Figure 10b), during which a jet of gas 103 discharges into the volume where the particles 58 are retained. It is thought that this jet 103 causes mixing of the gas and particles to create a gas-particle cloud that is quite uniform (see Figure 10c). Thus, when the downstream membrane 102 ruptures at a higher pressure, the particles 58 are already entrained in a cloud and a more uniform spread of particles is obtained. The delay time caused by the difference in rupturing pressure of the two membranes is sufficient to allow gas-particle mixing and a cloud to form and thereby overcome possible effects of gravity or attraction between the particles which cause the particles to bunch together (e.g. at the lowest point in the device) before rupture of the downstream membrane 102. Beneficially, a distance (e.g. a distance greater than one membrane radius) should separate the two membranes to allow for a clean membrane rupture and good mixing.

As a further modification, the particles 58 could initially be located upstream of the upstream membrane 101. When the upstream membrane 101 ruptures, the gas flowing into the space between the membranes carries the particles 58 with it and mixing is thus effected to produce a cloud in the same way as described above.

### Embodiment 7

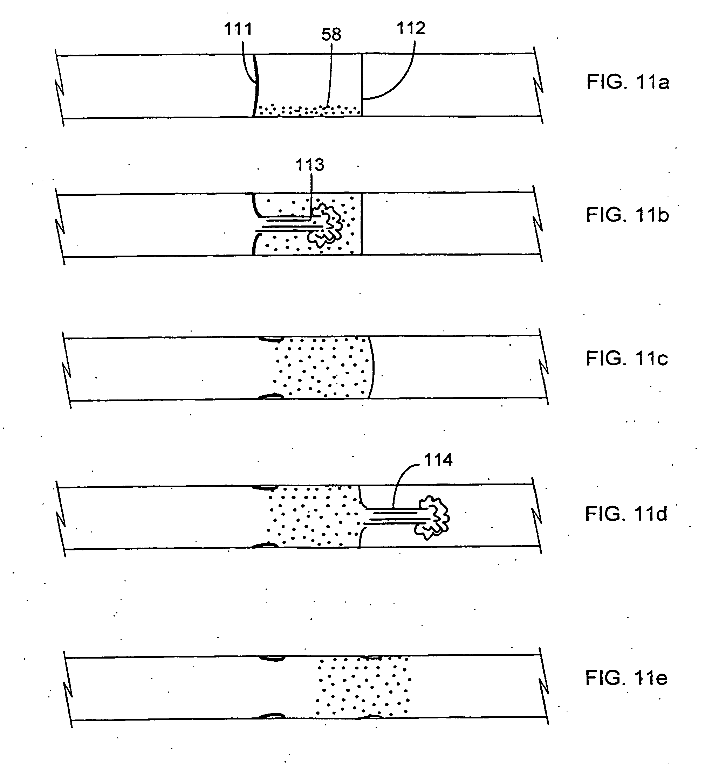

A further possible particle entrainment approach will now be described with reference to Figures 11a to 11e. Once again, the particles 58 are initially located between two membranes, however the upstream membrane 111 now has a higher rupture pressure than the downstream membrane 112 (see Figure 11a). As the upstream membrane 111 ruptures over a short but finite time, a jet of gas 113 mixes the particles as it fills the volume, and increases the local pressure (see Figures 11b and 11c). The downstream membrane 112 ruptures during or immediately after the rupture time of the upstream membrane 111 as a result of its lower rupture pressure. The new jet 114 (see Figure 11d) created by this process is weaker than jet 113, and the membrane 112 opens over a shorter rupture time than membrane 111 and results in a more controlled entrainment process (rupture time is determined in part by the unnecessary excess of pressure present in opening the membrane).

### Embodiment 8

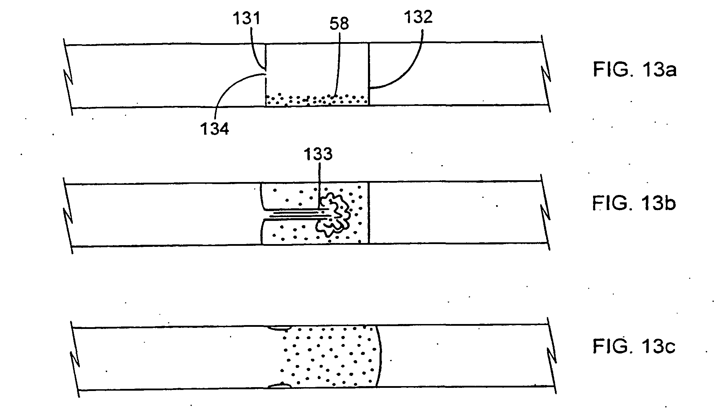

Figures 12a to 12c show stages in operation of an eighth embodiment of the invention, designed to aid particle mixing.

As can be seen from Figure 12a, a transfer duct 121 is provided to create a gas channel linking the driver duct to the volume between two membranes 123 and 124. The transfer duct is itself provided with a membrane 122 which ideally has a bursting pressure lower than that of membrane 123.

In operation, gas is fed to the driver chamber and enters the transfer duct. Membrane 122 ruptures when the gas reaches its predetermined bursting level. When membrane 122 ruptures, gas travels along the transfer duct and jets into the space between the membranes 123 and 124 (see Figure 12b). This causes mixing of the gas and particles 58 to create a cloud of gas and particles between the membranes The membrane 123 then bursts as does membrane 124, the timing being determined by the relative bursting pressures. It can be seen that the transfer duct serves to provide a jet of gas into the volume of particles to cause mixing before membranes 123 and 124 have ruptured.

The membrane 122 is not essential and may be dispensed with, especially if the transfer duct has a very small cross-sectional area so as to be effectively decoupled from the driver duct 51.

It is generally necessary for the transfer duct 121 to have a cross-sectional area smaller than the driver duct 51 to ensure that the driver gas does not completely bypass membrane 123 by flowing completely down the transfer duct 121. Further, membrane 122 may have a bursting pressure slightly higher than, or the same as, membrane 123. In this case, the side jet provided by the transfer duct 121 will be provided shortly after or at the same time as the jet provided by the opening of membrane 123.

One or more transfer ducts 121 may be provided in the device and one or some of these transfer ducts could be routed to provide side jets in volumes not initially housing particles.

A preferable modification of the above is shown in Figures 13a to 13c of the accompanying drawings. In this modification, the transfer duct consists of a small aperture 134 in the upstream closure means 131. Thus, when a gaseous pressure is exposed to the upstream closure means 131, some gas 133 is routed through the small aperture 134 which acts as a transfer duct prior to membrane rupture. This causes a similar mixing effect to that provided by separate, tubular, transfer ducts.

The upstream membrane 131 has an aperture 134 having a size small enough to prevent any particles escaping. The aperture is preferably circular and centred on the upstream membrane 131. Alternatively, the transfer duct may be provided by a membrane which is scored so as to burst in two stages; a centrally scored part would firstly rupture to allow a jet of gas 133 to enter the volume between the two membranes and then the rest of the membrane ruptures allowing a quasi-steady gas flow to be established.

The transfer duct 134 may ideally be provided by one or more pin-pricks in the membrane 131 or the central part of the membrane may be comprised of a series of leaf-like flaps which open when a gaseous pressure is applied thereto. Any means which allows gas to enter the volume between the upstream and downstream membrane before the upstream membrane ruptures fully is suitable.

Although in the description above the closure means has taken the form of a rupturable membrane, any other suitable rapidly openable closure means could alternatively be used, such as a non-membrane cassette.

The divergent nozzle 54 could have a simple profile or one that is contoured to ensures that the nozzle gas flow is uniform and free of oblique shocks. In this case, the parallel and uniform nozzle exit flow also sets up a nominally flat impingement shock and a more uniform impingement region.

The invention will still operate satisfactorily if the divergent nozzle 54 is dispensed with completely. In such a case, the gas undergoes a rapid expansion at the downstream end of the duct section 52. This case is equivalent to an under-expanded nozzle operation with a starting process in the downstream free jet which is analogous to the above starting process. Thus, a starting process of sorts is created even in the absence of a divergent nozzle and the concept of the invention is still applicable to devices having no divergent nozzle.

## Claims

1. A needleless injection device comprising:
a driver chamber (51) arranged, in use, to contain a charge of pressurised gas;
a duct section (52) connected to said driver chamber (51) to receive gas therefrom;
closure means (53) for preventing the flow of gas from said driver chamber to said duct section until said closure means (53) is opened; and
a dose of particles (58) positioned within the device in the region of said closure means (53) **characterised by**;
said device being so constructed and arranged that upon opening of said closure means (53), a primary shock wave (31) is produced to travel along said duct section (52) in a downstream direction and a substantially quasi-steady gas flow is established in said duct section upstream of said primary shock wave (31), said dose of particles (58) being substantially wholly entrained in said substantially quasi-steady flow to be accelerated thereby and expelled from the device.

2. A needleless injection device according to claim 1, wherein the device is arranged so that said primary shock wave (31) initiates a transient starting process upon reaching the downstream end of the duct section.

3. A needleless injection device according to claim 1 or 2, wherein said closure means (53) is positioned at the downstream extent of said driver chamber (51).

4. A needleless injection device according to any one of claims 1 to 3, wherein said driver chamber (51) is pre-charged with pressurised gas.

5. A needleless injection device according to any one of claims 1 to 3, further comprising a source (55) of gaseous fluid, said driver chamber (51) being fluidly connected to said source (55) and arranged to be provided with said charge of pressurised gas by said source upon opening of the fluid connection (56) therebetween.

6. A needleless injection device according to claim 5, wherein said fluid connection consists of a bleed hole (56) of a size small enough substantially to decouple said driver chamber (51) from said source (55) of gaseous fluid upon opening of said closure means (53).

7. A needleless injection device according to any one of claims 1 to 6, wherein said duct section (52) comprises a tube of substantially constant cross-sectional area.

8. A needleless injection device according to any one of claims 1 to 7, in which said particles (58) are positioned upstream of said closure means (53).

9. A needleless injection device according to any one of claims 1 to 8, wherein said duct section (52) includes substantially no convergent portion therein downstream of said closure means (53).

10. A needleless injection device according to any one claims 1 to 9, further comprising a divergent nozzle portion (54) positioned downstream of said duct section (52).

11. A needleless injection device according to claim 10, wherein said divergent nozzle portion (54) has an inlet cross-sectional area (A₁) and an exit cross-sectional area (Aₑ), said areas being chosen in accordance with the total driver chamber pressure at which said device is arranged to operate so that, in use, the gas flow in said divergent portion (54) is substantially correctly expanded when said particles (58) pass through said divergent portion.

12. A needleless injection device according to claim 10 or 11, wherein said divergent nozzle portion (54) has an internal contour such that substantially no oblique shock waves are formed in said substantially quasi-steady flow.

13. A needleless injection device according to any one of claims 10 to 12, wherein said divergent nozzle portion (54) is contoured such as to cause any expansion downstream of the duct section (52) to provide a generally radially uniform particle distribution at the exit of the divergent portion (54) and a generally radially uniform particle velocity distribution, with a substantially parallel velocity of particles and gas exiting the device.

14. A needleless injection device according to any one of claims 10 to 13, further comprising a spacer positioned at the downstream end of the device, the spacer being constructed so as to space a target plane (41) downstream of the divergent nozzle portion (54) exit with a clearance sufficient to allow:
a substantially normal shock (43) wave to be positioned downstream of the exit of said divergent nozzle portion (54); so that
said normal shock (43) interacts, in use, with the gas and particle jet from said device to provide a substantially controlled and uniform gas stagnation region which decelerates the particles to a generally uniform velocity as they impact the target plane.

15. A needleless injection device according to any one of claims 1 to 14, wherein said driver chamber (51) comprises a substantially constant area tube.

16. A needleless injection device according to any one claims 1 to 15, wherein said driver chamber (51) comprises a convergence at its downstream end, positioned upstream of said closure means (53).

17. A needleless injection device according to any one claims of claims 1 to 16, wherein said closure means comprises a rupturable membrane (53) arranged to open by rupturing.

18. A needleless injection device according to claim 17, wherein said rupturable membrane (53) is arranged to rupture in a controlled way due to an indentation on, or scoring of, the membrane (53) surface.

19. A needleless injection device according to any one claims 1 to 18, wherein said device contains a further closure means (101).

20. A needleless injection device according to claim 19, wherein said further closure means (101) is positioned in said driver chamber (51) upstream of said particles (58).

21. A needleless injection device according to claim 19 or 20, wherein said further closure means (101) comprises a rupturable membrane (101) arranged to open by rupturing.

22. A needleless injection device according to claim 21, wherein said rupturable membrane (101) is arranged to rupture in a controlled way due to an indentation on, or scoring of, its surfaces.

## Patentansprüche

1. Nadellose Injektionsvorrichtung mit:
einer Treiberkammer (51), welche bei Gebrauch so ausgelegt ist, dass sie eine Druckgasladung enthält;
einem mit der Treiberkammer (51) verbundenen Kanalabschnitt (52) zur Aufnahme von Gas von dieser;
einem Verschlussmittel (53) für das Verhindern des Strömens von Gas von der Treiberkammer zu dem Kanalabschnitt, bis das Verschlussmittel (53) geöffnet wird; und
einer Dosis von Partikeln (58), welche in der Vorrichtung in dem Bereich des Verschlussmittels (53) angeordnet sind, **gekennzeichnet dadurch, dass**
die Vorrichtung so konstruiert und ausgelegt ist, dass bei Öffnen des Verschlussmittels (53) eine primäre Druckwelle (31) erzeugt wird, um sich entlang des Kanalabschnitts (52) in Stromabwärtsrichtung fortzubewegen, und ein im Wesentlichen quasi gleichmäßiger Gasstrom in dem Kanalabschnitt stromaufwärts der primären Druckwelle (31) aufgebaut wird, wobei die Dosis an Partikeln (58) im Wesentlichen vollständig in dem im Wesentlichen quasi gleichmäßigen Strom mitgeführt wird, um durch beschleunigt und aus der Vorrichtung ausgestoßen zu werden.

2. Nadellose Injektionsvorrichtung nach Anspruch 1, **dadurch gekennzeichnet, dass** die primäre Druckwelle (31) bei Erreichen des stromabwärts gelegenen Endes des Kanalabschnitts einen vorübergehenden Startprozess einleitet.

3. Nadellose Injektionsvorrichtung nach Anspruch 1 oder 2, **dadurch gekennzeichnet, dass** das Verschlussmittel (53) an der stromabwärts gelegenen Ausdehnung der Treiberkammer (51) angeordnet ist.

4. Nadellose Injektionsvorrichtung nach einem der Ansprüche 1 bis 3, **dadurch gekennzeichnet, dass** die Treiberkammer (51) mit Druckgas vorbeladen ist.

5. Nadellose Injektionsvorrichtung nach einem der Ansprüche 1 bis 3, welche weiterhin eine Quelle (55) gasförmigen Fluids umfasst, wobei die Treiberkammer (51) mit der Quelle (55) fluidverbunden und so ausgelegt ist, dass sie bei Öffnen der Fluidverbindung (56) dazwischen mit der Ladung Druckgas durch die Quelle versorgt wird.

6. Nadellose Injektionsvorrichtung nach Anspruch 5, **dadurch gekennzeichnet, dass** die Fluidverbindung aus einem Zapfloch 56) von so kleiner Abmessung besteht, dass die Treiberkammer (51) bei Öffnen des Verschlussmittels (53) von der Quelle (55) gasförmigen Fluids im Wesentlichen entkuppelt wird.

7. Nadellose Injektionsvorrichtung nach einem der Ansprüche 1 bis 6, **dadurch gekennzeichnet, dass** der Kanalabschnitt (52) ein Rohr vom im Wesentlichen konstanter Querschnittfläche umfasst.

8. Nadellose Injektionsvorrichtung nach einem der Ansprüche 1 bis 7, **dadurch gekennzeichnet, dass** die Partikel (58) stromaufwärts des Verschlussmittels (53) angeordnet sind.

9. Nadellose Injektionsvorrichtung nach einem der Ansprüche 1 bis 8, **dadurch gekennzeichnet, dass** der Kanalabschnitt (52) darin im Wesentlichen keinen zusammenlaufenden Bereich stromabwärts des Verschlussmittels (53) aufweist.

10. Nadellose Injektionsvorrichtung nach einem der Ansprüche 1 bis 9, welche weiterhin einen stromabwärts des Kanalabschnitts (52) angeordneten auseinander laufenden Düsenteil (54) umfasst.

11. Nadellose Injektionsvorrichtung nach Anspruch 10, **dadurch gekennzeichnet, dass** der auseinander laufende Düsenteil (54) eine Einlassquerschnittfläche (A1) und eine Austrittquerschnittfläche (A2) aufweist, wobei die Flächen entsprechend dem gesamten Treiberkammerdruck gewählt werden, bei welchem die Vorrichtung zum Betrieb ausgelegt ist, so dass sich bei Gebrauch der Gasstrom in dem auseinander laufenden Teil (54) im Wesentlichen richtig entspannt, wenn die Partikel (58) den auseinander laufenden Teil passieren.

12. Nadellose Injektionsvorrichtung nach Anspruch 10 oder 11, **dadurch gekennzeichnet, dass** der auseinander laufende Düsenteil (54) eine solche Innenkontur aufweist, dass in dem im Wesentlichen quasi gleichmäßigen Strom im Wesentlichen keine quer verlaufenden Druckwellen gebildet werden.

13. Nadellose Injektionsvorrichtung nach einem der Ansprüche 10 bis 12, **dadurch gekennzeichnet, dass** der auseinander laufende Düsenteil (54) so konturiert ist, dass eine Entspannung stromabwärts des Kanalabschnitts (52) eine im Allgemeinen radial gleichmäßige Partikelverteilung am Austritt des auseinander laufenden Teils (54) und eine im Allgemeinen radial gleichmäßige Partikelgeschwindigkeitsverteilung mit einer im Wesentlichen parallelen Geschwindigkeit von aus der Vorrichtung austretenden Partikeln und Gas liefert.

14. Nadellose Injektionsvorrichtung nach einem der Ansprüche 10 bis 13, welche weiterhin einen am stromabwärts gelegenen Ende der Vorrichtung angeordneten Abstandshalter umfasst, wobei der Abstandshalter so konstruiert ist, dass bei einem hinreichenden Abstand eine Zielebene (41) stromabwärts des Austritts des auseinander laufenden Düsenteils (54) so beabstandet wird, dass:
eine im Wesentlichen senkrechte Druckwelle (43) stromabwärts des Austritts des auseinander laufenden Düsenteils (54) positioniert werden kann; so dass
die senkrechte Druckwelle (43) bei Gebrauch mit dem Gas- und Partikelstrahl aus der Vorrichtung in Wechselwirkung tritt, um einen im Wesentlichen gesteuerten und gleichmäßigen Gasstagnationsbereich zu liefern, welcher die Partikel auf eine im Allgemeinen gleichmäßige Geschwindigkeit abbremst, wenn sie auf die Zielebene treffen.

15. Nadellose Injektionsvorrichtung nach einem der Ansprüche 1 bis 16, **dadurch gekennzeichnet, dass** die Treiberkammer (51) ein Rohr von im Wesentlichen konstanter Fläche umfasst.

16. Nadellose Injektionsvorrichtung nach einem der Ansprüche 1 bis 15, **dadurch gekennzeichnet, dass** die Treiberkammer (51) an ihrem stromabwärts gelegenen Ende eine stromaufwärts des Verschlussmittels (53) angeordnete Konvergenz umfasst.

17. Nadellose Injektionsvorrichtung nach einem der Ansprüche 1 bis 16, **dadurch gekennzeichnet, dass** das Verschlussmittel eine so ausgelegte reißbare Membran (53) umfasst, dass diese durch Reißen öffnet.

18. Nadellose Injektionsvorrichtung nach Anspruch 17, **dadurch gekennzeichnet, dass** die reißbare Membran (53) so ausgelegt ist, dass sie aufgrund einer Einkerbung an bzw. einem Einritzen der Oberfläche der Membran (53) in gesteuert Weise- reißt.

19. Nadellose Injektionsvorrichtung nach einem der Ansprüche 1 bis 18, **dadurch gekennzeichnet, dass** die Vorrichtung ein weiteres Verschlussmittel (101) enthält.

20. Nadellose Injektionsvorrichtung nach Anspruch 19, **dadurch gekennzeichnet, dass** das weitere Verschlussmittel (101) stromaufwärts der Partikel (58) in der Treiberkammer (51) angeordnet ist.

21. Nadellose Injektionsvorrichtung nach Anspruch 19 oder 20, **dadurch gekennzeichnet, dass** das weitere Verschlussmittel (101) eine so ausgelegte reißbare Membran (101) umfasst, dass diese durch Reißen öffnet.

22. Nadellose Injektionsvorrichtung nach Anspruch 21, **dadurch gekennzeichnet, dass** die reißbare Membran (101) so ausgelegt ist, dass sie aufgrund einer Einkerbung an bzw. einem Einritzen ihrer Oberflächen in gesteuerter Weise reißt.

## Revendications

1. Dispositif d'injection sans aiguille, comprenant :
une chambre de dispositif d'actionnement (51) agencée, lors de l'utilisation, pour contenir une charge de gaz comprimé ;
une section de conduit (52) connectée à ladite chambre de dispositif d'actionnement (51) pour recevoir un gaz à partir de celle-ci ;
des moyens de fermeture (53) pour empêcher l'écoulement de gaz de ladite chambre de dispositif d'actionnement à ladite section de conduit jusqu'à ce que lesdits moyens de fermeture (53) soient ouverts ; et
une dose de particules (58) positionnée à l'intérieur du dispositif dans la région desdits moyens de fermeture (53), **caractérisé en ce que** :
ledit dispositif est construit et agencé de telle sorte que, lors de l'ouverture desdits moyens de fermeture (53), une onde de choc primaire (31) soit produite de façon à se déplacer le long de ladite section de conduit (52) dans une direction aval et qu'un écoulement de gaz sensiblement quasi-continu soit établi dans ladite section de conduit en amont de ladite onde de choc primaire (31), ladite dose de particules (58) étant sensiblement totalement entraînée dans ledit écoulement sensiblement quasi-continu afin d'être accélérée par celui-ci et expulsée du dispositif.

2. Dispositif d'injection sans aiguille selon la revendication 1, dans lequel le dispositif est agencé de telle sorte que ladite onde de choc primaire (31) déclenche un processus de démarrage transitoire lors de l'atteinte de l'extrémité aval de la section de conduit.

3. Dispositif d'injection sans aiguille selon la revendication 1 ou 2, dans lequel lesdits moyens de fermeture (53) sont positionnés dans la zone aval de ladite chambre de dispositif d'actionnement (51).

4. Dispositif d'injection sans aiguille selon l'une quelconque des revendications 1 à 3, dans lequel ladite chambre de dispositif d'actionnement (51) est préchargée en gaz comprimé.

5. Dispositif d'injection sans aiguille selon l'une quelconque des revendications 1 à 3, comprenant de plus une source (55) de fluide gazeux, ladite chambre de dispositif d'actionnement (51) étant reliée de manière fluidique à ladite source (55) et agencée de façon à se voir délivrer ladite charge de gaz comprimé par ladite source lors de l'ouverture de la connexion de fluide (56) entre celles-ci.

6. Dispositif d'injection sans aiguille selon la revendication 5, dans lequel ladite connexion de fluide est constituée par un trou d'évacuation (56) ayant une taille suffisamment petite pour découpler sensiblement ladite chambre de dispositif d'actionnement (51) de ladite source (55) de fluide gazeux lors de l'ouverture desdits moyens de fermeture (53).

7. Dispositif d'injection sans aiguille selon l'une quelconque des revendications 1 à 6, dans lequel ladite section de conduit (52) comprend un tube ayant une surface de section transversale sensiblement constante.

8. Dispositif d'injection sans aiguille selon l'une quelconque des revendications 1 à 7, dans lequel lesdites particules (58) sont positionnées en amont desdits moyens de fermeture (53).

9. Dispositif d'injection sans aiguille selon l'une quelconque des revendications 1 à 8, dans lequel ladite section de conduit (52) ne contient sensiblement aucune partie convergente à l'intérieur de celle-ci en aval desdits moyens de fermeture (53).

10. Dispositif d'injection sans aiguille selon l'une quelconque des revendications 1 à 9, comprenant de plus une partie de buse divergente (54) positionnée en aval de ladite section de conduit (52).

11. Dispositif d'injection sans aiguille selon la revendication 10, dans lequel ladite partie de buse divergente (54) a une surface de section transversale d'entrée (A₁) et une surface de section transversale de sortie (Aₑ), lesdites surfaces étant choisies en fonction de la pression totale de chambre de dispositif d'actionnement à laquelle ledit dispositif est agencé pour fonctionner, de telle sorte que, lors de l'utilisation, l'écoulement de gaz dans ladite partie divergente (54) soit élargi sensiblement correctement lorsque lesdites particules (58) traversent ladite partie divergente.

12. Dispositif d'injection sans aiguille selon la revendication 10 ou 11, dans lequel ladite partie de buse divergente (54) a un profil intérieur tel que sensiblement aucune ondes de choc obliques ne soient formées dans ledit écoulement sensiblement quasi-continu.

13. Dispositif d'injection sans aiguille selon l'une quelconque des revendications 10 à 12, dans lequel ladite partie de buse divergente (54) est profilée de façon à faire en sorte que toute expansion en aval de la section de conduit (52) produise une distribution de particules globalement radialement uniforme au niveau de la sortie de la partie divergente (54), et une distribution de vitesse de particules globalement radialement uniforme, avec une vitesse sensiblement parallèle des particules et du gaz sortant du dispositif.

14. Dispositif d'injection sans aiguille selon l'une quelconque des revendications 10 à 13, comprenant de plus un élément d'espacement positionné à l'extrémité aval du dispositif, l'élément d'espacement étant construit de façon à espacer un plan cible (41) en aval de la sortie de partie de buse divergente (54), avec un espacement suffisant pour permettre :
à une onde de choc sensiblement normal (43) d'être positionnée en aval de la sortie de ladite partie de buse divergente (54) ; de telle sorte que :
ledit choc normal (43) interagisse, lors de l'utilisation, avec le jet de gaz et de particules venant dudit dispositif de façon à produire une région de stagnation de gaz sensiblement contrôlée et uniforme qui décélère les particules à une vitesse globalement uniforme lorsqu'elles heurtent le plan cible.

15. Dispositif d'injection sans aiguille selon l'une quelconque des revendications 1 à 14, dans lequel ladite chambre de dispositif d'actionnement (51) comprend un tube de surface sensiblement constante.

16. Dispositif d'injection sans aiguille selon l'une quelconque des revendications 1 à 15, dans lequel ladite chambre de dispositif d'actionnement (51) comprend une convergence à son extrémité aval, positionnée en amont desdits moyens de fermeture (53).

17. Dispositif d'injection sans aiguille selon l'une quelconque des revendications 1 à 16, dans lequel lesdits moyens de fermeture comprennent une membrane pouvant être rompue (53) agencée de façon à s'ouvrir par rupture.

18. Dispositif d'injection sans aiguille selon la revendication 17, dans lequel ladite membrane pouvant être rompue (53) est agencée de façon à se rompre d'une façon contrôlée grâce à une indentation, ou à une entaille, de la surface de la membrane (53).

19. Dispositif d'injection sans aiguille selon l'une quelconque des revendications 1 à 18, dans lequel ledit dispositif contient d'autres moyens de fermeture (101).

20. Dispositif d'injection sans aiguille selon la revendication 19, dans lequel lesdits autres moyens de fermeture (101) sont positionnés dans ladite chambre de dispositif d'actionnement (51) en amont desdites particules (58).

21. Dispositif d'injection sans aiguille selon la revendication 19 ou 20, dans lequel lesdits autres moyens de fermeture (101) comprennent une membrane pouvant être rompue (101) agencée de façon à s'ouvrir par rupture.

22. Dispositif d'injection sans aiguille selon la revendication 21, dans lequel ladite membrane pouvant être rompue (101) est agencée de façon à se rompre d'une façon contrôlée grâce à une indentation, ou une entaille, de ses surfaces.
